# NOUVEAU FASCICULE DE BREVET EUROPEEN

(11) **EP 1 782 791 B2**
(45) Date de publication et mention de la décision concernant l'opposition: **23.01.2019**
(45) Mention de la délivrance du brevet: 07.07.2010
(21) Numéro de dépôt: 06122117.2
(22) Date de dépôt: 11.10.2006
(51) Int. Cl.: A61K 8/49, A61K 8/41, A61K 8/92, A61Q 5/12

(54) **Composition cosmétique comprenant un tensioactif cationique,un corps gras liquide et un ester de sorbitan**
Kosmetische Zusammensetzung enthaltend ein kationisches Tensid,einen flüssigen Fettkörper und einen Sorbitanester
Cosmetic composition comprising a cationic surfactant,a liquid fatty material and a sorbitan ester

(30) Priorité: 28.10.2005 FR 0553284
(43) Date de publication de la demande: 09.05.2007
(73) Titulaire: L'Oréal, 75008 Paris (FR)
(72) Inventeur: Fack, Géraldine, 92300 Levallois (FR); Mahe, Véronique, 78740 Vaux s/Seine (FR)
(74) Mandataire: Dodin, Catherine

(56) Documents cités:
- EP-A- 1 132 079
- FR-A- 2 847 831
- US-A1- 2004 151 746
- US-A1- 2004 223 938

## Description

La présente invention est relative à une composition cosmétique notamment de conditionnement des cheveux comprenant au moins un tensioactif cationique, au moins un ester de sorbitan oxyéthyléné et au moins un corps gras liquide et à un procédé de traitement cosmétique des matières kératiniques en particulier les cheveux.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà proposé pour le traitement des matières kératiniques et en particulier des cheveux, des compositions cosmétiques comprenant tensioactifs cationiques.

De telles compositions présentent cependant des inconvénients tels que des problèmes de rinçabilité, des problèmes de stabilité, des difficultés de répartition sur les matières kératiniques ainsi que des propriétés cosmétiques insuffisantes.

On a déjà préconisé dans les compositions pour le lavage ou le soin des matières kératiniques telles que les cheveux l'utilisation de polymères cationiques, de silicones cationiques ou de tensioactifs cationiques pour faciliter le démêlage des cheveux et pour leur communiquer douceur et souplesse. L'usage des polymères cationiques ou des cations dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion interfibres affectant le coiffage.

De plus les soins utilisés pour les cheveux très sensibilisés peuvent être insuffisant pour traiter les pointes qui sont le plus souvent très abîmées.

En résumé, il s'avère que les compositions cosmétiques actuelles de conditionnement, ne donnent pas complètement satisfaction. Ainsi, on cherche à obtenir des compositions cosmétiques ayant de très bonnes propriétés cosmétiques en particulier sur des cheveux très sensibilisés.

La demanderesse a maintenant découvert que l'association d'un tensioactif cationique, d'au moins un ester de sorbitan oxyéthyléné particulier et d'au moins un corps gras liquide permet de remédier à ces inconvénients.

Les cheveux traités avec cette composition sont lisses, se démêlent facilement, sont brillants, souples, individualisés et ont un toucher doux et sans résidus. Les cheveux ont un aspect naturel et non chargé. Le lissage est homogène des racines aux pointes. Les pointes présentent moins de fourches.

De plus, ces effets sont rémanents au cours du temps.

La titulaire a découvert que l'adjonction d'ester de sorbitan faiblement oxyéthyléné, permettait en plus de réduire de façon surprenante les réactions d'inconfort (démangeaisons, rougeurs ...), notamment au niveau du cuir chevelu, des compositions contenant des tensioactifs susceptibles de provoquer ce type de réactions lorsqu'ils sont utilisés seuls.

Ainsi, selon la présente invention, il est maintenant proposé de nouvelles compositions cosmétiques, comprenant, dans un milieu aqueux cosmétiquement acceptable, au moins un tensioactif cationique, au moins un ester de sorbitan oxyéthyléné et d'acide gras en C₈-C₂₄, saturé, linéaire ou ramifié et ayant un nombre de moles d'oxyde d'éthylène inférieur ou égal à 10 et au moins un corps gras non siliconé liquide à une température de 25°C et à pression atmosphérique (1 atm), le ou les corps gras non siliconés liquides étant présent dans une quantité de 0,1 à 5% en poids par rapport au poids total de la composition.

Un autre objet de l'invention consiste en un procédé de traitement cosmétique des matières kératiniques, en particulier les cheveux mettant en oeuvre la composition susvisée.

L'invention a encore pour objet l'utilisation de ladite composition comme après-shampoing.

D'autres objets, caractéristiques, aspects et avantages de l'invention apparaîtront encore plus clairement à la lecture de la description et des divers exemples qui suivent.

Par cheveux sensibilisés, on comprend selon la présente invention, des cheveux ayant subi des agressions extérieures physiques (lumière, chaleur, ondes...), des agressions mécaniques (brushing, peignages ou brossages répétés...) ou des agressions chimiques (coloration d'oxydation, décoloration, permanente, défrisage...). Les compositions selon l'invention sont particulièrement efficaces sur les cheveux sensibilisés par des agressions chimiques.

Par « au moins un », on comprendra « un ou plusieurs », C'est-à-dire un, deux, trois ou plus.

Par "milieu cosmétiquement acceptable", on entend un milieu compatible avec toutes les matières kératiniques telles que la peau, les cheveux, les ongles, les cils, les sourcils, les lèvres et toute autre zone du corps et du visage.

Les acides gras saturés des esters de sorbitan et d'acide gras saturé en C₈-C₂₄, ont un nombre de moles d'oxyde d'éthylène inférieur ou égal à 10. Les acides gras sont notamment l'acide laurique et l'acide stéarique, et de préférence l'acide laurique.

On utilise de préférence des mono-esters d'acide gras en C8-C24 et de sorbitan oxyethylene. Le nombre moyen de moles d'oxyde d'éthylène va plus particulièrement de 3 à 8 moles d'oxyde d'éthylène et en particulier est égal à 4.

Les esters de sorbitan oxyéthyléné et d'acide gras en C₈-C₂₄, saturé, linéaire ou ramifié et ayant un nombre de moles d'oxyde d'éthylène inférieur ou égal à 10 présentent généralement la formule suivante :

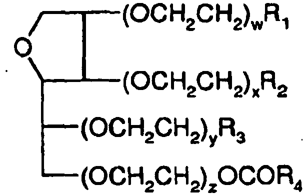

Dans laquelle w, x et y sont des nombres allant de 0 à 9, de préférence égaux à 0,

Dans laquelle z est un nombre allant de 1 à 10, allant de préférence de 3 à 8 et plus particulièrement égale à 4.

Dans laquelle la somme de w + x + y + z est une valeur moyenne inférieure ou égale à 10, allant de préférence de 3 à 8 et plus particulièrement égale à 4.

R1, R2 et R3, identiques ou différents désignent H ou C(=O)R5 et de préférence H.

R4 désigne un radical alkyl saturé, linéaire ou ramifié, ayant de 8 à 24 atomes de carbone et plus particulièrement de 8 à 18 atomes de carbone.

R5 désigne un radical alkyl saturé ayant de 8 à 30 atomes de carbone et plus particulièrement de 8 à 18 atomes de carbone.

Les esters de sorbitan préférés sont le mono-laurate de sorbitan oxyéthyléné avec 4 moles d'oxyde d'éthylène (4OE) ou polysorbate 21 et le mono-stéarate de sorbitan oxyéthyléné avec 4 moles d'oxyde d'éthylène (4OE) ou polysorbate 61.

Le polysorbate 21 est particulièrement préféré et est notamment commercialisé sous la dénomination TWEEN 21 par la société UNIQEMA.

Selon la présente invention, l'ester de sorbitan oxyétyléné peut être présent dans la composition cosmétique dans des proportions allant de 0,1 à 10%, préférentiellement de 0,5 à 8 % en poids et plus particulièrement de 1 à 6% en poids par rapport au poids total de ladite composition.

La composition selon l'invention comprend un ou plusieurs tensioactifs cationiques bien connus en soi, tels que les sels d'amines grasses primaires, secondaires ou tertiaires, éventuellement polyoxyalkylénées, les sels d'ammonium quaternaire, et leurs mélanges.

Selon l'invention, les tensioactifs cationiques sont non polymériques.

A titre d'amines grasses, on peut notamment citer les alkyl amido amines telles que par exemple les alkyl (C8-C30) amido dialkyl (C1-C6)amines et en particulier la stéaramido propyl diméthylamine (MACKINE 301 proposé par MAC INTYRE).

A titre de sels d'ammonium quaternaire, on peut notamment citer, par exemple :
- ceux qui présentent la formule générale (V) suivante : 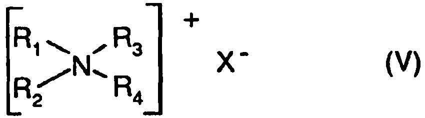 dans laquelle les symboles R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes. Les radicaux aliphatiques sont par exemple choisis parmi les radicaux alkyle en C1-C30, , alcoxy, polyoxyalkylène (C₂-C₆), alkylamide, alkyl(C₁₂-C₂₂)amidoalkyle(C₂-C₆), alkyl(C₁₂-C₂₂)acétate, hydroxyalkyle, comportant environ de 1 à 30 atomes de carbone ; X est un anion choisi dans le groupe des halogénures, phosphates, acétates, lactates, alkyl (C₂-C₆)sulfates, alkyl- ou alkylaryl-sulfonates ;
- les sels d'ammonium quaternaire de l'imidazoline comme, par exemple, ceux de formule (VI) suivante : 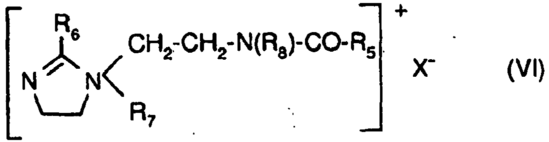 dans laquelle R₅ représente un radical alcényle ou alkyle comportant de 8 à 30 atomes de carbone par exemple dérivés des acides gras du suif ou du coprah, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 2002), le Quaternium-87 (CTFA 2002) ou le Quaternium-83 (CTFA 2002) commercialisés sous les dénominations "VARISOFT®" W575PG par la société GOLDSCHMIDT,
- les sels de diammonium quaternaire de formule (VII) : 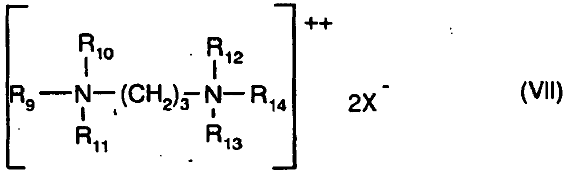 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrates, éthylsulfates et méthylsulfates. De tels sels de diammonium quaternaire comprennent notamment le dichlorure de propanesuif diammonium ;
- les sels d'ammonium quaternaire comprenant au moins une fonction ester, tels que ceux de formule (VIII) suivante : 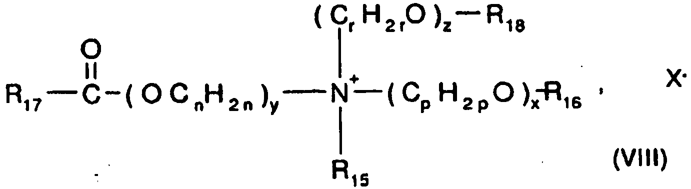 dans laquelle :
   R₁₅ est choisi parmi les radicaux alkyles en C₁-C₆ et les radicaux hydroxyalkyles ou dihydroxyalkyles en C₁-C₆ ;
   R₁₆ est choisi parmi :
      - le radical 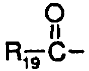
      - les radicaux R₂₀ hydrocarbonés en C₁-C₂₂, linéaires ou ramifiés, saturés ou insaturés,
      - l'atome d'hydrogène,
   R₁₇ est choisi parmi :
      - le radical 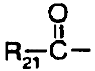
      - les radicaux R₂₂ hydrocarbonés en C₁-C₆, linéaires ou ramifiés, saturés ou insaturés,
      - l'atome d'hydrogène,
         R₁₇, R₁₉ et R₂₁, identiques ou différents, sont choisis parmi les radicaux hydrocarbonés en C₇-C₂₁, linéaires ou ramifiés, saturés ou insaturés ;
         r, n et p, identiques ou différents, sont des entiers valant de 2 à 6;
         y est un entier valant de 1 à 10 ;
         x et z, identiques ou différents, sont des entiers valant de 0 à 10;
         X est un anion simple ou complexe, organique ou inorganique ;
         sous réserve que la somme x + y + z vaut de 1 à 15, que lorsque x vaut 0 alors R₁₆ désigne R₂₀ et que lorsque z vaut 0 alors R₁₈ désigne R₂₂.

Les radicaux alkyles R₁₅ peuvent être linéaires ou ramifiés, et plus particulièrement linéaires.

De préférence, R₁₅ désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R₁₆ est un radical R₂₀ hydrocarboné, il peut être long et avoir de 12 à 22 atomes de carbone, ou court et avoir de 1 à 3 atomes de carbone.

Lorsque R₁₈ est un radical R₂₂ hydrocarboné, il a de préférence 1 à 3 atomes de carbone.

Avantageusement, R₁₇, R₁₉ et R₂₁, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.

Avantageusement, y est égal à 1.

De préférence, r, n et p, identiques ou différents, valent 2 ou 3, et encore plus particulièrement sont égaux à 2.

L'anion X- est de préférence un halogénure (chlorure, bromure ou iodure) ou un alkyl(C1-C4)sulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

On utilise plus particulièrement dans la composition selon l'invention, les sels d'ammonium de formule (IV) dans laquelle :
- R₁₅ désigne un radical méthyle ou éthyle,
- x et y sont égaux à 1 ;
- z est égal à 0 ou 1 ;
- r, n et pt sont égaux à 2 ;
- R₁₆ est choisi parmi :
- le radical 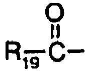
- les radicaux méthyle, éthyle ou hydrocarbonés en C₁₄-C₂₂,
- l'atome d'hydrogène;
- R₁₈ est choisi parmi :
- le radical 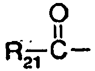
- l'atome d'hydrogène ;
- R₁₇, R₁₉ et R₂₁, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires.

On peut citer par exemple les composés de formule (VIII) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxyéthyl-méthylammonium, de monoacyloxyéthyl-hydroxyéthyl-diméthylammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.

De tels composés sont par exemple commercialisés sous les dénominations DEHYQUART® par la société COGNIS, STEPANQUAT® par la société STEPAN, NOXAMIUM® par la société CECA, REWOQUAT® WE 18 par la société REWO-GOLDSCHMIDT.

La composition selon l'invention peut contenir de préférence un mélange de sels de mono-, di- et triester d'ammonium quaternaire avec une majorité en poids de sels de diester.

Comme mélange de sels d'ammonium, on peut utiliser par exemple le mélange comprenant 15 à 30 % en poids de méthylsulfate d'acyloxyéthyl-dihydroxyéthyl-méthylammonium, 45 à 60% de méthylsulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium comprenant au moins une fonction ester décrits dans les brevets US-A-4874554 et US-A-4137180.

Parmi les sels d'ammonium quaternaire mentionnés ci-dessus, on préfère utiliser ceux répondant à la formule (V). On peut notamment citer d'une part, les chlorures de tétraalkylammonium comme, par exemple, les chlorures de dialkyldiméthylammonium ou d'alkyltriméthylammonium dans lesquels le radical alkyle comporte environ de 12 à 22 atomes de carbone, en particulier les chlorures de béhényltriméthylammonium, de distéaryldiméthylammonium, de cétyltriméthylammonium, de benzyldiméthylstéarylammonium ou encore, d'autre part, le chlorure de palmitylamidopropyltriméthylammonium ou le chlorure de stéaramidopropyldiméthyl-(myristyl acétate)-ammonium correspondant au QUATERNIUM-70 (CTFA 2002) commercialisé sous la dénomination CERAPHYL® 70 par la société ISP.

Les tensioactifs cationiques particulièrement préférés dans la composition de l'invention sont choisis parmi les sels d'ammonium quaternaire, et en particulier parmi le chlorure de béhényltriméthylammonium, le chlorure de cétyltriméthylammonium, le quaternium-83, le quaternium-87, le chlorure de béhénylamidopropyl 2,3-dihydroxypropyl diméthyl ammonium, le chlorure de palmitylamidopropyltriméthylammonium et la stéaramidopropyldiméthylamine.

La composition selon l'invention comprend de préférence le ou les tensioactifs cationiques en une quantité allant de 0,01 à 10 % en poids, de préférence de 0,05 à 4 % en poids par rapport au poids total de la composition et plus particulièrement de 0,1 à 3% en poids.

Par corps gras non siliconé, on entend au sens de la présente invention tout corps organique huileux ne comportant pas d'atomes de silicium dans sa structure élémentaire, comportant au moins une chaîne carbonée comportant au moins 10 atomes de carbone et dont la solubilité dans l'eau à 25°C (1atm) est inférieure à 0,1% en poids.

Les corps gras liquides non siliconés sont notamment choisis parmi les alcools gras oxyéthylénés ou non, les esters gras, les huiles végétales, les huiles hydrocarbonées et leurs mélanges.

Les alcools gras selon l'invention sont de préférence ramifiés et/ou insaturés, et comportent de 12 à 40 atomes de carbone.

Les alcools gras présentent de préférence la structure R-OH, dans laquelle R désigne de préférence un groupement alkyle ramifié en C12-C24 ou alkényle en C12-C24. R peut être substitué par un ou plusieurs groupements hydroxy. De préférence, R ne contient pas de groupement hydroxy.

A titre d'exemple on peut citer l'alcool oléique, l'alcool isocétylique, l'alcool isostéarylique, l'octyl dodécanol, le 2-éthylhexyl dodécanol et leurs mélanges.

L'alcool gras peut représenter un mélange d'alcools gras, ce qui signifie que dans un produit commercial peuvent coexister plusieurs espèces d'alcools gras, sous forme d'un mélange.

Par alcool gras oxyalkyléné selon l'invention, on entend tout alcool gras de structure suivante : 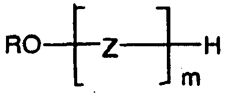 dans laquelle :
R désigne un radical saturé ou insaturé, linéaire ou ramifié, comportant de 8 à 40 atomes de carbone et de préférence de 8 à 30,
Z représente un radical oxyéthyléné (i) et/ou oxypropyléné (!!)1 et (ii)2 de formules respectives suivantes :

   **-CH₂-CH₂-O-** **(i)**

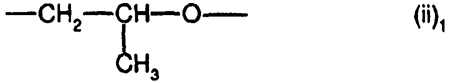

   **-CH₂-CH₂-CH₂-O-** **(ii)₂**
m représente le nombre de groupements oxyde d'éthylène (i) et/ou oxyde de propylène (ii)1 ou (ii)2, allant de 1 à 20 et de préférence de 2 à 10.

Des alcools gras oxyalkylénés liquides particulièrement préférés selon l'invention sont des alcools gras, saturés ou non, linéaires, comportant de 10 à 20 atomes de carbone et 2 à 8 groupements oxyde d'éthylène.

Comme composés de type alcool gras oxyalkyléné, on peut notamment citer les produits commercialisés suivants :
Mergital LM2 (COGNIS) [alcool laurique 2 OE] ;
Empilan KA 2.5/90FL (ALBRIGHT & WILSON) et Mergital BL309 (COGNIS) [alcool décylique 3 OE] ;
Empilan KA 5/90 FL (ALBRIGHT & WILSON) et Mergital BL589 (COGNIS) [alcool décylique 5 OE] ;
Emulgin 05 (COGNIS) [alcool oléocétylique 5 OE] ;
Witconol APM (GOLDSCHMIT) [alcool myristique 3 OP] ;

De préférence, les alcools gras de l'invention sont non oxyalkylénés. Ces alcools gras peuvent être des constituants des cires animales ou végétales.

Les esters gras liquides sont des esters liquides d'acide carboxylique comportant de préférence au moins 10 atomes de carbone et plus particulièrement de 10 à 40 atomes de carbone, tels que par exemple, l'huile de Purcellin (octanoate de stéaryle), le myristate d'isopropyle, le palmitate d'isopropyle, le stéarate de butyle, le laurate d'hexyle, l'adipate de diisopropyle, l'isononanoate d'isononyle, le palmitate de 2-éthylhexyle, le lactate de 2-octyldodécyle, le néopentanoate d'isostéaryle, le néopentanoate de tridécyle, le néopentanoate d'isocétyle et le néopentanoate d'isoarachidyle et leurs mélanges.

Les huiles végétales peuvent être choisies parmi l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de cade, la cire liquide de jojoba, l'huile de calophyllum et leurs mélanges.

Comme huiles hydocarbonées, on peut citer les huiles minérales notamment l'huile de paraffine et l'huile de vaseline, les isoparaffines tels que les polyisobutylènes, les polydécènes ; et leurs mélanges.

Le ou les corps gras liquides sont présents dans la composition à une teneur allant de 0,1 à 5%, de préférence de 0,5 à 3% en poids du poids total de la composition.

La composition selon l'invention peut contenir éventuellement d'autres tensioactifs que les tensioactifs cationiques.

Les tensioactifs additionnels peuvent être présents en une quantité allant de 0,1 % à 10% en poids environ, de préférence de 0,5% à 8% et encore plus préférentiellement de 1% à 5%, par rapport au poids total de la composition.

Les tensioactifs additionnels sont de préférence choisis parmi les tensioactifs non ioniques.

Les agents tensioactifs non-ioniques sont des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools gras polyéthoxylés, polypropoxylés ou polyglycérolés, les alpha-diols gras polyéthoxylés, polypropoxylés ou polyglycérolés, les alkylphénols gras polyéthoxylés, polypropoxylés ou polyglycérolés, ou les acides gras polyéthoxylés, polypropoxylés ou polyglycérolés, tous ces composés ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine.

Les compositions selon l'invention sont de préférence des compositions non lavantes (non détergentes), elles comprennent de préférence moins de 4% en poids de tensioactifs détergents notamment anioniques et plus particulièrement moins de 1% en poids par rapport au poids total de la composition et encore mieux elles ne contiennent pas de tensioactifs détergent.

La composition selon l'invention peut comprendre en outre au moins un agent conditionneur additionnel.

Cet agent conditionneur est notamment choisi parmi les silicones, les polymères cationiques, les esters gras carboxyliques solides, et leurs mélanges.

Les silicones utilisables conformément à l'invention peuvent être solubles ou insolubles dans la composition, et elles peuvent être en particulier des polyorganosiloxanes insolubles dans la composition de l'invention. Elles peuvent se présenter sous forme d'huiles, de cires, de résines ou de gommes. Elles peuvent être utilisées pures ou en émulsion, en dispersion ou en microémulsion.

Les organopolysiloxanes sont définis plus en détail dans l'ouvrage de Walter NOLL "Chemistry and Technology of Silicones" (1968) Academic Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
1.
   (i) les silicones cycliques comportant de 3 à 7 atomes de silicium et, de préférence, 4 à 5. Il s'agit, par exemple, de l'octaméthylcyclotétra-siloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA, ainsi que leurs mélanges. On peut également citer les cyclocopolymères du type diméthylsiloxane/méthylalkylsiloxane, tel que la "SILICONE VOLATILE FZ 3109" commercialisée par la société UNION CARBIDE, de structure chimique : 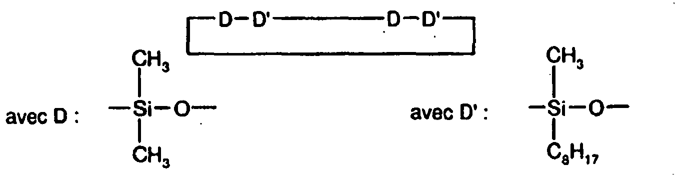 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
   (ii) les silicones volatiles linéaires ayant 2 à 9 atomes de silicium et possédant une viscosité inférieure ou égale à 5.10⁻⁶m²/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

Parmi les silicones non volatiles, on peut notamment citer les polyalkylsiloxanes, les polyarylsiloxanes, les polyalkylarylsiloxanes, les gommes et les résines de silicones, les polyorganosiloxanes modifiés par des groupements organofonctionnels ainsi que leurs mélanges.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone-copolyol commercialisé par la société DOW CORNING sous la dénomination DC 1248 ou les huiles SILWET® L 722, L 7500, L 77, L 711 de la société UNION CARBIDE et l'alkyl(C₁₂)-méthicone-copolyol commercialisée par la société DOW CORNING sous la dénomination Q2 5200 ;
- des groupements aminés substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄;
- des groupements thiols, comme les produits commercialisés sous les dénominations "GP 72 A" et "GP 71" de GENESEE;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT ;
- des groupements hydroxylés, comme les polyorganosiloxanes à fonction hydroxyalkyle décrits dans la demande de brevet français FR-A-85 16334 ;
- des groupements acyloxyalkyle tels que, par exemple, les polyorganosiloxanes décrits dans le brevet US-A-4957732 ;
- des groupements anioniques du type acide carboxylique comme, par exemple, dans les produits décrits dans le brevet EP 186 507 de la société CHISSO CORPORATION, ou du type alkyl-carboxylique comme ceux présents dans le produit X-22-3701^{E} de la société SHIN-ETSU ; 2-hydroxyalkylsulfonate ; 2-hydroxyalkylthiosulfate tels que les produits commercialisés par la société GOLDSCHMIDT sous les dénominations "ABIL® S201 "et"ABIL® S255" ;
- des groupements hydroxyacylamino, comme les polyorgano-siloxanes décrits dans la demande EP 342 834. On peut citer, par exemple, le produit Q2-8413 de la société DOW CORNING.

A titre d'exemples de silicones, on utilise de préférence les polydiméthylsiloxanes, les polyalkylarylsiloxanes, les polydiméthylsiloxanes à groupements aminés ou alcoxylés.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression « polymère cationique » désigne tout polymère contenant des groupements cationiques et/ou des groupements ionisables en groupements cationiques.

Parmi tous les polymères cationiques susceptibles d'être utilisés dans le cadre de la présente invention, on préfère mettre en oeuvre les dérivés d'éther de cellulose quaternaires tels que les produits commercialisés sous la dénomination « JR 400 » par la société AMERCHOL, les cyclopolymères, en particulier les homopolymères de sel de diallyldiméthylammonium et les copolymères de sel de diallyldiméthylammonium et d'acrylamide en particulier les chlorures, commercialisés sous les dénominations « MERQUAT 100 », « MERQUAT 550 » et « MERQUAT S » par la société NALCO, les polysaccharides cationiques et plus particulièrement les gommes de guar modifiées par du chlorure de 2,3-époxypropyl triméthylammonium commercialisées par exemple sous la dénomination « JAGUAR C13S » par la société MEYHALL, les homopolymères et les copolymères éventuellement réticulés de sel de (méth)acryloyloxyéthyltriméthylammonium, vendus par la société CIBA en solution à 50% dans de l'huile minérale sous les dénominations commerciales SALCARE SC92 (copolymère réticulé du chlorure de méthacryloyloxyéthyltriméthylammonium et de l'acrylamide) et SALCARE SC95 (homopolymère réticulé du chlorure de méthacryloyloxyéthyl triméthylammonium).

On peut également utiliser les polymères qui sont constitués de motifs récurrents répondant à la formule : 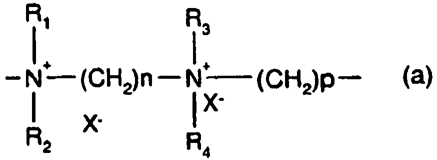 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.

Les agents conditionneurs additionnels sont contenus de préférence dans la composition selon l'invention en une quantité allant de 0,01 % à 20 % en poids, mieux encore allant de 0,1 % à 10 % en poids et plus particulièrement allant de 0,3 % à 5 % en poids par rapport au poids total de la composition.

Le milieu cosmétiquement acceptable est de préférence aqueux et peut être comprendre de l'eau ou un mélange d'eau et d'un solvant cosmétiquement acceptable tel qu'un alcool inférieur en C₁-C₄, par exemple l'éthanol, l'isopropanol, le tertio-butanol, le n-butanol ; les polyols comme le propylèneglycol, la glycérine ; les éthers de polyols ; les alcanes en C₅-C₁₀ ; et leurs mélanges.. Les solvants sont de préférence choisis parmi la glycérine et le propylène glycol.

Le milieu cosmétiquement acceptable notamment aqueux représente de préférence de 30 à 98% en poids par rapport au poids total de la composition.

Les solvants sont de préférence présents dans des concentrations allant de 0,5 à 30% en poids par rapport au poids total de la composition.

Le pH des compositions de l'invention est compris de préférence de 2 à 8, de préférence de 3 à 7.

Les compositions selon l'invention peuvent également contenir des additifs classiques bien connus dans la technique, tels que des polymères anioniques, non ioniques ou amphotères, des épaississants non polymériques comme des acides ou des électrolytes, des opacifiants, des nacrants, des vitamines, des provitamines telles que le panthénol, des parfums, des colorants, des particules organiques ou minérales, des conservateurs, des agents de stabilisation du pH, des agents antipelliculaires comme de la piroctone olamine, des agents antichute des cheveux..

L'homme de métier veillera à choisir les éventuels additifs et leur quantité de manière à ce qu'ils ne nuisent pas aux propriétés des compositions de la présente invention.

Ces additifs sont présents dans la composition selon l'invention en une quantité allant de 0 à 20 % en poids par rapport au poids total de la composition.

Les compositions de l'invention peuvent se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Elles peuvent être utilisées, par exemple, comme après-shampoings, soins, masques de soin profond, lotions ou crèmes de traitement du cuir chevelu. Ces compositions peuvent être rincées ou non rincées.

Selon un mode de réalisation préféré de l'invention, la composition peut être utilisée comme après-shampoing, en particulier sur des cheveux sensibilisés. Cet après-shampooing peut être rincé ou non rincé et de préférence rincé.

Les compositions cosmétiques selon l'invention peuvent se présenter sous forme de gel, de lait, de crème, d'émulsion, de lotions fluides ou épaissies ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

La présente invention concerne également un procédé de traitement cosmétique des matières kératiniques telles que par exemple la peau ou les cheveux qui consiste à appliquer une quantité efficace d'une composition cosmétique telle que décrite ci-dessus, sur les matières kératiniques, à effectuer un éventuel rinçage après un éventuel temps de pose.

Le rinçage s'effectue par exemple avec de l'eau.

Ainsi, ce procédé selon l'invention permet le traitement, le conditionnement, le soin des cheveux ou de toute autre matière kératinique.

Les exemples suivants illustrent la présente invention et ne doivent être considérés en aucune manière comme limitant l'invention.

### EXEMPLES 1 A 5

On a préparé les compositions suivantes d'après-shampooing:
1. Dans la cuve de fabrication introduire la totalité de l'eau, ajouter les composés hydrosolubles à l'exception du ou des esters de sorbitan oxyéthyléné. Chauffer jusqu'à 80°C sous agitation raclante jusqu'à complète dissolution.
2. Dans une cuve annexe introduire les composés non hydrosolubles hormis les parfums et les silicones Chauffer jusqu'à 80°C
3. Introduire le contenu de l'annexe et les éventuelles silicones dans la cuve de fabrication et émulsionner 10 mn sous forte agitation turbine et raclante en maintenant la température. Puis commencer le refroidissement.
4. à 30°C introduire l'ester de sorbitan oxyéthyléné et les parfum sous agitation raclante.

### - Exemple 1 d'après shampooings à rincer :

| | En g MA |
|---|---|
| Alcool laurique oxyéthyléne (4 OE) (BRIJ 30 de UNIQUEMA) | 1 |
| Huile de palme (Refined palm oil de welch holme & clark) | 1 |
| Chlorure de cétyl triméthyl ammonium à 25% MA (ARQUAD 16-25 lo de AKZO NOBEL) | 0,45 |
| Stéaryl amido propyl diméthylamine (MACKINE 301 de MAC INTYRE) | 0,75 |
| Mono laurate de sorbitane oxyéthyléné à 4 OE (TWEEN 21 de UNIQUEMA) | 4 |
| Polydiméthylsiloxane à groupement aminoéthyl iminopropyl (DOW CORNING 939 EMULSION) | 0,63 |
| Parfum | qs |
| Conservateurs | qs |
| Eau | qsp 100 g |

### - Exemple 2 d'après shampooings non rincé :

| | en g MA |
|---|---|
| Monoisostéarate de polyéthylene glycol (PRISORINE 3644 de UNIQUEMA) | 0,25 |
| Chlorure de béhényl triméthyl ammonium à 80%MA (GENAMIN KDMP de CLARIANT) | 0,20 |
| Huile d'avocat (LIPOVOL A de LIPO CHEMICALS) | 0,65 |
| Cire liquide de jojoba | 0,65 |
| Ethanol | 14,3 |
| 2.4 - diamino pyridine -3- oxyde (MEXORYL SAG de CHIMEX) | 1,5 |
| Cyclopentadiméthylsiloxane (MIRASIL CM 5 de RHODIA) | 0,45 |
| Mono laurate de sorbitane oxyéthyléné avec 4 moles d'oxyde d'éthylène (4 OE) (TWEEN 21 de UNIQUEMA) | 0,5 |
| Parfum, conservateurs | qs |
| Eau | Qsp 100 g |

### - Exemple 3 d'après shampooings non rincé antipelliculaire :

| | En g MA |
|---|---|
| Monoisostéarate de polyéthylène glycol | 0,25 |
| (PRISORINE 3644 de UNIQUEMA) | |
| Chlorure de béhényl triméthyl ammonium à 80%MA (GENAMIN KDMP de CLARIANT) | 0,2 |
| Huile d'avocat (LIPOVOL A de LIPO CHEMICALS) | 0,65 |
| Cire liquide de jojoba (Pure jojoba oil de jojoba israel) | 0,65 |
| Ethanol | 14,3 |
| Piroctone olamine (OCTOPIROX de CLARIANT) | 0,1 |
| Cyclopentadiméthylsiloxane MIRASIL CM 5 de RHODIA | 0,45 |
| Mono laurate de sorbitan oxyéthyléné à 4 OE (TWEEN 21 de UNIQUEMA) | 0,5 |
| Parfum, conservateurs | qs |
| Eau | Qsp 100g |

### - Exemple 4 d'après shampooings à rincer:

| | **En g MA** |
|---|---|
| Alcool laurique oxyéthyléné (4 OE) (BRIJ 30 de UNIQUEMA) | 3 |
| Huile de palme (Refined palm oil de welch holme & clark) | 1 |
| Phosphate de diamidon de mais hydroxypropylé prégélatinisé (STRUCTURE ZEA de NATIONAL STARCH) | 6 |
| Chlorure de cétyl triméthyl ammonium à 25% MA (ARQUAD 16-25 lo de AKZO NOBEL) | 0,45 |
| Stéaryl amido propyl diméthylamine (MACKINE 301 de MAC INTYRE) | 0,75 |
| Mono laurate de sorbitan oxyéthyléné à 4 OE (TWEEN 21 de UNIQUEMA) | 4 |
| Polydiméthylsiloxane à groupements aminoéthyl Iminopropyl à 35% MA (DOW CORNING 939 EMULSION) | 0,63 |
| Parfum | qs |
| Conservateur | qs |
| Kaolinite (SUPREME de YMERIS) | 3 |
| Eau | qsp 100 g |

### - Exemple 5 d'après shampooings rincé ou non rincé :

| | en g MA |
|---|---|
| Perhydrosqualène (squalane de kishimoto) | 1,85 |
| Chlorure de béhényl triméthyl ammonium à 80%MA (GENAMIN KDMP de CLARIANT) | 0,31 |
| Cyclopentadiméthyl siloxane (MIRASIL CM 5 de RHODIA) | 2,1 |
| Propylène glycol | 2 |
| Cire liquide de jojoba (pure jojoba oil de jojoba israel) | 1,05 |
| Mono laurate de sorbitane oxyéthyléné à 4 moles d'oxyde d'éthylène (TWEEN 21 de UNIQUEMA) | 5 |
| Eau | qsp 100 g |

Ces compositions ont été appliquées sur des cheveux très sensibilisés. Les propriétés cosmétiques (démêlage, lissage, souplesse, brillance) sont excellentes et homogènes des racines aux pointes des cheveux.
Entre deux applications, les cheveux restent doux, souples et lisses.

## Revendications

1. Composition cosmétique, comprenant, dans un milieu aqueux cosmétiquement acceptable, au moins un tehsioactif cationique, au moins un ester de sorbitan oxyéthyléné et d'acide gras en C₈-C₂₄, saturé, linéaire ou ramifié et ayant un nombre de moles d'oxyde d'éthylène inférieur ou égal à 10 et au moins un corps gras non siliconé liquide à une température de 25°C, le corps gras liquide étant présent dans une quantité de 0,1 à 5% en poids par rapport au poids total de la composition, le corps gras liquide étant choisi parmi l'alcool oléique, l'alcool isocétylique, l'alcool isostéarylique, l'octyl dodécanol, le 2-éthyl hexyl dodécanol, l'huile de Purcellin (2-éthyl hexanoate de stéaryle), le myristate d'isopropyle, le palmitate d'isopropyle, le stéarate dé butyle, le laurate d'hexyle, l'adipate de diisopropyle, l'isononanoate d'isononyle, le palmitate de 2-éthylhexyle, le lactate de 2-octyldodécyle, le néopentanoate d'isostéaryle, le néopentanoate de tridécyle, le néopentanoate d'isocétyle, le néopentanoate d'isoarachidyle, les huiles végétales, les huiles hydrocarbonées.

2. Composition selon la revendication précédente, **caractérisée en ce que** les tensioactifs cationiques sont choisis parmi les sels d'amines grasses primaires, secondaires ou tertiaires, éventuellement polyoxyalkylénées, les sels d'ammonium quaternaire et leurs mélanges.

3. Composition selon la revendication 2, **caractérisée en ce que** les sels d'ammonium quaternaire sont choisis parmi :
- ceux qui présentent là formule générale (V) suivante : 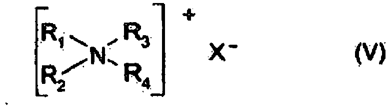 dans laquelle les symboles R₁ à R₄, qui peuvent être identiques ou différents, représentent un radical aliphatique, linéaire ou ramifié, comportant de 1 à 30 atomes de carbone, ou un radical aromatique tel que aryle ou alkylaryle ; X⁻ est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline ;
- les sels de diammonium quaternaire de formule (VII) : 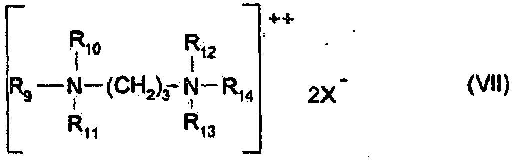 dans laquelle R₉ désigne un radical aliphatique comportant environ de 16 à 30 atomes de carbone, R₁₀, R₁₁, R₁₂, R₁₃ et R₁₄, identiques ou différents sont choisis -parmi l'hydrogène ou un radical allyle comportant de 1 à 4 atomes de carbone, et X⁻ est un anion choisi dans le groupe des halogénures, acétates, phosphates, nitrates et méthylsulfates ;
- les sels d'ammonium quaternaire comprenant au moins une fonction ester.

4. Composition selon la revendication 3, **caractérisée en ce que** les sels d'ammonium quaternaire de l'imidazoline sont choisis parmi ceux de formule (VI) suivante : 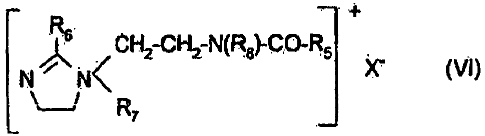 dans laquelle R₅ représente un radical alcényle ou alkyle comportant de 8 à 30 atomes de carbone par exemple dérivés des ecides gras du suif, R₆ représente un atome d'hydrogène, un radical alkyle en C₁-C₄ ou un radical alcényle ou alkyle comportant de 8 à 30 atomes de carbone. R₇ représente un radical alkyle en C₁-C₄, R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène.

5. Composition selon l'une quelconque des revendications précédentes, **caractérisée en ce que** les tensloactifs cationiques sont choisis parmi le chlorure de béhényltriméthylammonium, le chlorure de cétyltriméthylammonium, le Quaternium-83, le Quaternium-87, le chlorure de béhénylamidopropyl 2,3-dihydroxypropyl diméthyl ammonium et le chlorure de palmitylamidopropyltriméthylammonlum et la stéaramidopropyldiméthylamine.

6. Composition selon l'une quelconque des revendications précédentes, **caractérisée en ce que** les tensioactifs cationiques sont contenus en une quantité allant de 0,01 à 10 % en poids, de préférence dé 0,05 à 4 % en poids par rapport au poids total de la composition.

7. Composition selon l'une quelconque des revendications précédentes, **caractérisée en ce que** les acides gras des esters de sorbitan et d'acide gras en C₈-C₂₄, ayant un nombre de moles d'oxyde d'éthylène inférieur ou égal à 10 ont de 8 à 18 atomes de carbone.

8. Composition selon la revendication précédente, **caractérisée en ce que** les acides gras saturés des esters de sorbitan et d'acide gras en C₈-C₂₄, sont choisis parmi l'acide laurique et l'acide stéarique, de préférence l'acide laurique.

9. Composition selon l'une quelconque des revendications précédentes, **caractérisée en ce que** les esters de sorbitan oxyéthyléné sont choisis des mono-esters d'acide gras en C8-C24 et de sorbitan oxyéthyléné.

10. Composition selon l'une quelconque des revendications précédentes **caractérisée en ce que** le nombre de moles d'oxyde d'éthylène va de 3 à 8 moles d'oxyde d'éthylène et en particulier est égal à 4.

11. Composition selon l'une quelconque des revendications précédentes, **caractérisée en ce que** les esters de sorbitan oxyéthyléné sont choisis parmi le monolaurate de sorbitan oxyéthyléné avec 4 moles d'oxyde d'éthylène (40E) et le monostéarate de sorbitan oxyéthyléné avec 4 moles d'oxyde d'éthylène (40E).

12. Composition selon l'une quelconque des revendications précédentes, **caractérisée en ce que** l'ester de sorbitan oxyéthyléné est le mono-laurate de sorbitan oxyéthyléné avec 4 moles d'oxyde d'éthylène (40E).

13. Composition selon l'une quelconque des revendications précédentes, **caractérisée en ce que** l'ester de sorbitan oxyéthyléné est présent dans la composition dans des proportions allant de 0,1 à 10%, et préférentiellement de 0,5 à 8 % en poids par rapport au poids total de ladite composition,

14. Composition selon l'une des revendications précédentes, **caractérisée en ce que** les hulles végétales sont choisies parmi l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, la cire liquide de jojoba, l'huile de cade, l'huile de calophyllum et leurs mélanges.

15. Composition selon l'une des revendications précédentes, **caractérisée en ce que** les huiles hydocarbonées sont choisies parmi l'huile de paraffine, l'huile de vaseline, les isoparaffines et leurs mélanges.

16. Composition selon l'une quelconque des revendications précédentes, **caractérisée par le fait que** le corps gras liquide est présent à une concentration allant de 0,5 à 3% en poids du poids total de la composition.

17. Composition selon l'une quelconque des revendications précédentes, **caractérisée en ce qu'**elle comprend en outre au moins un agent conditionneur additionnel.

18. Composition selon la revendication 17, **caractérisée en ce que** l'agent conditionneur additionnel est choisi parmi les silicones, les polymères cationiques, les esters gras carboxyliques solides, et leurs mélanges.

19. Composition selon l'une quelconque des revendications précédentes, **caractérisée en ce que** le milieu aqueux cosmétiquement acceptable est constitué par de l'eau ou par un mélange d'eau et d'un solvant cosmétiquement acceptable.

20. Composition selon la revendication 19, **caractérisée en ce que** le solvant cosmétiquement acceptable est choisi parmi les alcools inférieur en C₁-C₄ tel que l'éthanol, l'isopropanol, le tertio-butanol, le n-butanol; les alkylèneglycols comme le propylèneglycol, les éthers de polyol ; les alcanes en C₅-C₁₀ et leurs mélanges.

21. Composition selon l'une quelconque des revendications précédantes, **caractérisée en ce qu'**elle comprend en outre des additifs tels que des polymères anioniques, non Ioniques ou amphotères, des épaississants comme des acides ou des électrolytes, des opacifiants, des agents nacrants, des vitamines, des provitamines telles que le panthénol, des parfums, des colorants, des particules organiques ou minérales, des conservateurs, des agents de stabilisation du pH.

22. Composition selon l'une quelconque des revendications précédentes, **caractérisée par le fait qu'**elle se présente sous formé d'après-shampooing, de composition pour la permanente, le défrisage, la coloration ou la décoloration des cheveux, de composition à rincer à appliquer entre les deux étapes d'une permanente ou d'un défrisage.

23. Composition selon l'une quelconque des revendications précédentes, **caractérisée en ce qu'**elle est se présente sous la forme d'un après-shampoing à rincer.

24. Procédé de traitement cosmétique des matières kératiniques, telles que les cheveux, **caractérisé en ce qu'**il consiste à appliquer sur lesdites matières une quantité efficace d'une composition cosmétique selon l'une des revendications 1 à 23, puis à effectuer éventuellement un rinçage.

## Patentansprüche

1. Kosmetische Zusammensetzung, die in einem kosmetisch akzeptablen wässrigen Medium mindestens einen kationischen grenzflächenaktiven Stoff, mindestens einen Ester von ethoxyliertem Sorbitan und einer geradkettigen oder verzweigten, gesättigten C₈₋₂₄-Fettsäure mit einer Molzahl von Ethylenoxid von kleiner oder gleich 10 und mindestens eine bei einer Temperatur von 25°C flüssigen, nicht siliconierten Fettsubstanz enthält, wobei die flüssige Fettsubstanz in einer Menge von 0,1 bis 5 Gew.-%, bezogen auf das Gesamtgewicht der Zusammensetzung, enthalten ist und die flüssige Fettsubstanz unter Oleylalkohol, Isocetylalkohol, Isostearylalkohol, Octyldodecanol, 2-Ethylhexyldodecanol, Purcellinöl (Stearyl-2-ethylhexanoat), Isopropylmyristat, Isopropylpalmitat, Butylstearat, Hexyllaurat, Diisopropyladipat, Isononylisononanoat, 2-Ethylhexylpalmitat, 2-Octyldodecyllactat, Isostearylneopentanoat, Tridecylneopentanoat, Isocetylneopentanoat, Isoarachidylneopentanoat, pflanzlichen Ölen und Kohlenwasserstoffölen ausgewählt ist.

2. Zusammensetzung nach dem vorhergehenden Anspruch, **dadurch gekennzeichnet, dass** die kationischen grenzflächenaktiven Stoffe unter den Salzen von primären, sekundären oder tertiären Fettaminen, die gegebenenfalls polyalkoxyliert sind, quartären Ammoniumsalzen und deren Gemischen ausgewählt sind.

3. Zusammensetzung nach Anspruch 2, **dadurch gekennzeichnet, dass** die quartären Ammoniumsalze ausgewählt sind unter:
• quartären Ammoniumsalzen der folgenden allgemeinen Formel (V): 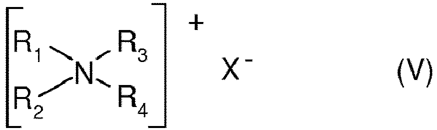 worin die Gruppen R₁ bis R₄, die gleich oder verschieden sein können, eine geradkettige oder verzweigte, aliphatische Gruppe mit 1 bis 30 Kohlenstoffatomen oder eine aromatische Gruppe, wie Aryl oder Alkylaryl, bedeuten; und X⁻ ein Anion ist, das unter den Halogeniden, Phosphaten, Acetaten, Lactaten, Alkyl(C₂₋₆)sulfaten, Alkyl- oder Alkylarylsulfonaten ausgewählt ist;
• quartären Ammoniumsalzen von Imidazolin;
• quartären Diammoniumsalzen der Formel (VII): 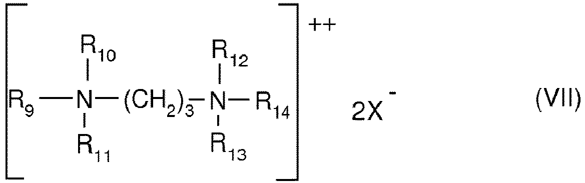 worin die Gruppe R₆ eine aliphatische Gruppe mit etwa 16 bis 30 Kohlenstoffatomen bedeutet, die Gruppen R₁₀, R₁₁, R₁₂, R₁₃ und R₁₄, die gleich oder verschieden sind, unter Wasserstoff oder einer Alkylgruppe mit 1 bis 4 Kohlenstoffatomen ausgewählt sind, und X⁻ ein Anion ist, das unter den Halogeniden, Acetaten, Phosphaten, Nitraten und Methylsulfaten ausgewählt ist;
• quartären Ammoniumsalzen, die mindestens eine Esterfunktion enthalten.

4. Zusammensetzung nach Anspruch 3, **dadurch gekennzeichnet, dass** die quartären Ammoniumsalze von Imidazolin unter den Verbindungen der folgenden Formel (VI) ausgewählt sind: 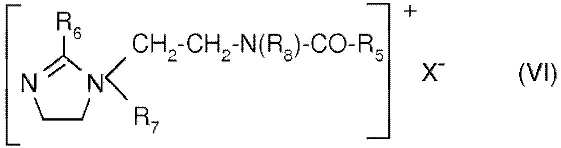 worin R₅ eine Alkenyl- oder Alkylgruppe mit 8 bis 30 Kohlenstoffatomen bedeutet, die beispielsweise von Fettsäuren aus Talg abgeleitet sind, R₆ ein Wasserstoffatom, C₁₋₄-Alkyl oder eine Alkenyl- oder Alkylgruppe mit 8 bis 30 Kohlenstoffatomen bedeutet, R₇ eine C₁₋₄-Alkylgruppe ist, R₈ ein Wasserstoffatom oder C₁₋₄-Alkyl bedeutet und X⁻ ein Anion ist, das unter den Halogeniden, Phosphaten, Acetaten, Lactaten, Alkylsulfaten, Alkyl- oder Alkylarylsulfonaten ausgewählt ist; die Gruppen R₅ und R₆ bedeuten vorzugsweise ein Gemisch aus Alkenyl- oder Alkylgruppen mit 12 bis 21 Kohlenstoffatomen, die beispielsweise von Talgfettsäuren abgeleitet sind, R₇ bedeutet Methyl und R₈ ist Wasserstoff.

5. Zusammensetzung nach einem der vorhergehenden Ansprüche, **dadurch gekennzeichnet, dass** die kationischen grenzflächenaktiven Stoffe unter Behenyltrimethylammoniumchlorid, Cetyltrimethylammoniumchlorid, Quaternium-83, Quaternium-87, Behenylamidopropyl-2,3-dihydroxypropyl-dimethylammoniumchlorid, Palmitylamidopropyltrimethylammoniumchlorid und Stearamidopropyldimethylamin ausgewählt sind.

6. Zusammensetzung nach einem der vorhergehenden Ansprüche, **dadurch gekennzeichnet, dass** die grenzflächenaktiven Stoffe in einem Menganteil von 0,01 bis 10 Gew.-% und vorzugsweise 0,05 bis 4 Gew.-%, bezogen auf das Gesamtgewicht der Zusammensetzung, enthalten sind.

7. Zusammensetzung nach einem der vorhergehenden Ansprüche, **dadurch gekennzeichnet, dass** die Fettsäuren der Ester von Sorbitan und einer C₈₋₂₄-Fettsäure mit einer Molzahl von Ethylenoxid von 10 oder darunter 8 bis 18 Kohlenstoffatome aufweisen.

8. Zusammensetzung nach dem vorhergehenden Anspruch, **dadurch gekennzeichnet, dass** die gesättigten Fettsäuren der Ester von Sorbitan und einer C₈₋₂₄-Fettsäure unter Laurinsäure und Stearinsäure und vorzugsweise Laurinsäure ausgewählt sind.

9. Zusammensetzung nach einem der vorhergehenden Ansprüche, **dadurch gekennzeichnet, dass** die ethoxylierten Sorbitanester unter den Monoestern einer C₈₋₂₄-Fettsäure und ethoxyliertem Sorbitan ausgewählt sind.

10. Zusammensetzung nach einem der vorhergehenden Ansprüche, **dadurch gekennzeichnet, dass** die Ethylenoxid-Molzahl im Bereich von 3 bis 8 mol Ethylenoxid liegt und insbesondere 4 beträgt.

11. Zusammensetzung nach einem der vorhergehenden Ansprüche, **dadurch gekennzeichnet, dass** die ethoxylierten Sorbitanester unter mit 4 mol Ethylenoxid (4 EO) ethoxyliertem Sorbitanmonolaurat und mit 4 mol Ethylenoxid (4 EO) ethoxyliertem Sorbitanmonostearat ausgewählt sind.

12. Zusammensetzung nach einem der vorhergehenden Ansprüche, **dadurch gekennzeichnet, dass** es sich bei dem ethoxylierten Sorbitanester um das mit 4 mol Ethylenoxid (4 EO) ethoxylierte Sorbitanmonolaurat handelt.

13. Zusammensetzung nach einem der vorhergehenden Ansprüche, **dadurch gekennzeichnet, dass** der ethoxylierte Sorbitanester in der Zusammensetzung in Mengenanteilen von 0,1 bis 10 % und vorzugsweise 0,5 bis 8 Gew.-%, bezogen auf das Gesamtgewicht der Zusammensetzung, enthalten ist.

14. Zusammensetzung nach einem der vorhergehenden Ansprüche, **dadurch gekennzeichnet, dass** die pflanzlichen Öle unter Süßmandelöl, Avocadoöl, Ricinusöl, Olivenöl, Jojobaöl, Sonnenblumenöl, Getreidekeimöl, Sesamöl, Erdnussöl, Traubenkernöl, Sojaöl, Rapsöl, Distelöl, Kopraöl, Maisöl, Haselnussöl, Sheabutter, Palmöl, Aprikosenkernöl, flüssigem Jojobawachs, Wacholderöl, Calophyllumöl und deren Gemischen ausgewählt ist.

15. Zusammensetzung nach einem der vorhergehenden Ansprüche, **dadurch gekennzeichnet, dass** die Kohlenwasserstofföle unter Paraffinöl, Vaselineöl, Isoparaffinen und deren Gemischen ausgewählt sind.

16. Zusammensetzung nach einem der vorhergehenden Ansprüche, **dadurch gekennzeichnet, dass** die flüssige Fettsubstanz in einer Konzentration von 0,5 bis 3 Gew.-% des Gesamtgewichts der Zusammensetzung vorliegt.

17. Zusammensetzung nach einem der vorhergehenden Ansprüche, **dadurch gekennzeichnet, dass** sie ferner mindestens ein ergänzendes Konditioniermittel enthält.

18. Zusammensetzung nach Anspruch 17, **dadurch gekennzeichnet, dass** das ergänzende Konditioniermittel unter den Siliconen, kationischen Polymeren, festen Fettsäureestern und deren Gemischen ausgewählt ist.

19. Zusammensetzung nach einem der vorhergehenden Ansprüche, **dadurch gekennzeichnet, dass** das kosmetisch akzeptable wässrige Medium aus Wasser oder aus einem Gemisch von Wasser und einem kosmetisch akzeptablen Lösungsmittel besteht.

20. Zusammensetzung nach Anspruch 19, **dadurch gekennzeichnet, dass** das kosmetisch akzeptable Lösemittel unter den niederen C₁₋₄-Alkoholen, wie Ethanol, Isopropanol, tert-Butanol, n-Butanol; Alkylenglycolen, wie Propylenglycol, Polyolethern; C₅₋₁₀-Alkanen und deren Gemischen ausgewählt ist.

21. Zusammensetzung nach einem der vorhergehenden Ansprüche, **dadurch gekennzeichnet, dass** sie ferner Additive enthält, wie beispielsweise anionische, nichtionische oder amphotere Polymere, Verdickungsmittel, wie Säuren oder Elektrolyte, Trübungsmittel, Perlglanzstoffe, Vitamine, Provitamine, wie Panthenol, Parfüms, Farbmittel, organische oder anorganische Partikel, Konservierungsmittel oder pH-Stabilisatoren.

22. Zusammensetzung nach einem der vorhergehenden Ansprüche, **dadurch gekennzeichnet, dass** sie in Form einer Pflegespülung, als Zusammensetzung für dauerhafte Verformungen, zum Entkräuseln, zum Färben oder zum Entfärben der Haare, als Zusammensetzung, die ausgespült wird und zwischen den beiden Schritten einer dauerhaften Verformung oder einer Entkräuselung aufgebracht wird, vorliegt.

23. Zusammensetzung nach einem der vorhergehenden Ansprüche, **dadurch gekennzeichnet, dass** sie in Form einer Pflegespülung vorliegt, die ausgespült wird.

24. Verfahren zur kosmetischen Behandlung von Keratinsubstanzen, wie Haaren, **dadurch gekennzeichnet, dass** es darin besteht, auf die Keratinsubstanzen eine wirksame Menge einer Zusammensetzung nach einem der Ansprüche 1 bis 22 aufzubringen und anschließend gegebenenfalls zu spülen.

## Claims

1. Cosmetic composition comprising, in a cosmetically acceptable aqueous medium, at least one cationic surfactant, at least one oxyethylenated sorbitan ester and of a saturated, linear or branched C₈-C₂₄ fatty acid with a number of moles of ethylene oxide of less than or equal to 10, and at least one non-silicone fatty substance liquid at a temperature of 25°C, the liquid fatty substance being present in an amount of from 0,1 to 5% by weight relative to the total weight of the composition, the liquid fatty substance being chosen from oleyl alcohol, isocetyl alcohol, isostearyl alcohol, octyldodecanol, 2-ethylhexyldodecanol, purcellin oil (stearyl 2-ethylhexanoate), isopropyl myristate, isopropyl palmitate, butyl stearate, hexyl laurate, diisopropyl adipate, isononyl isononanoate, 2-ethylhexyl palmitate, 2-octyldodecyl lactate, isostearyl neopentanoate, tridecyl neopentanoate, isocetyl neopentanoate, isoarachidyl neopentanoate, plant oils and hydrocarbon-based oils.

2. Composition according to the preceding claim, **characterized in that** the cationic surfactants are chosen from optionally polyoxyalkylenated primary, secondary or tertiary fatty amine salts and quaternary ammonium salts, and mixtures thereof.

3. Composition according to Claim 2, **characterized in that** the quaternary ammonium salts are chosen from:
- those having the general formula (V) below: 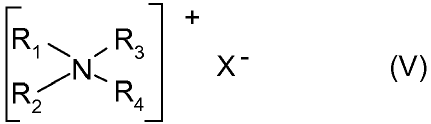 in which the symbols R₁ to R₄, which may be identical or different, represent a linear or branched aliphatic radical containing from 1 to 30 carbon atoms or an aromatic radical such as aryl or alkylaryl; X⁻ is an anion chosen from the group of halides, phosphates, acetates, lactates, (C₂-C₆) alkyl sulfates, alkylsulfonates and alkylarylsulfonates;
- quaternary ammonium salts of imidazoline;
- diquaternary ammonium salts of formula (VII): 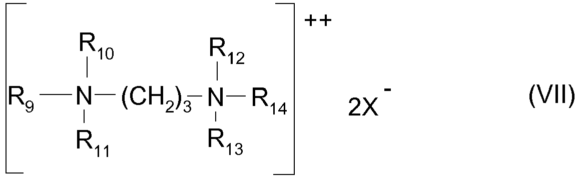 in which R₆ denotes an aliphatic radical containing from about 16 to 30 carbon atoms, R₁₀, R₁₁, R₁₂, R₁₃ and R₁₄, which may be identical or different, are chosen from hydrogen and an alkyl radical containing from 1 to 4 carbon atoms, and X⁻ is an anion chosen from the group of halides, acetates, phosphates, nitrates and methyl sulfates;
- quaternary ammonium salts comprising at least one ester function.

4. Composition according to Claim 3, **characterized in that** the quaternary ammonium salts of imidazoline are chosen from those of formula (VI) below: 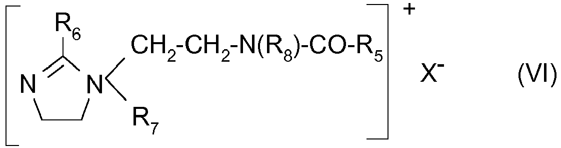 in which R₅ represents an alkenyl or alkyl radical containing from 8 to 30 carbon atoms, for example fatty acid derivatives of tallow, R₆ represents a hydrogen atom, a C₁-C₄ alkyl radical or an alkenyl or alkyl radical containing from 8 to 30 carbon atoms, R₇ represents a C₁-C₄ alkyl radical, R₈ represents a hydrogen atom or a C₁-C₄ alkyl radical, X⁻ is an anion chosen from the group of halides, phosphates, acetates, lactates, alkyl sulfates, alkylsulfonates and alkylarylsulfonates. Preferably, R₅ and R₆ denote a mixture of alkenyl or alkyl radicals containing from 12 to 21 carbon atoms, for example fatty acid derivatives of tallow, R₇ denotes methyl and R₈ denotes hydrogen.

5. Composition according to any one of the preceding claims, **characterized in that** the cationic surfactants are chosen from behenyltrimethylammonium chloride, cetyltrimethylammonium chloride, Quaternium-83, Quaternium-87, behenylamidopropyl-2,3-dihydroxypropyldimethylammonium chloride and palmitylamidopropyltrimethylammonium chloride and stearamidopropyldimethylamine.

6. Composition according to any one of the preceding claims, **characterized in that** the cationic surfactants are contained in an amount ranging from 0.01% to 10% by weight and preferably from 0.05% to 4% by weight relative to the total weight of the composition.

7. Composition according to any one of the preceding claims, **characterized in that** the fatty acids of the esters of sorbitan and of a C₈-C₂₄ fatty acid, with a number of moles of ethylene oxide of less than or equal to 10, contain from 8 to 18 carbon atoms.

8. Composition according to the preceding claim, **characterized in that** the saturated fatty acids of the esters of sorbitan and of a C₈-C₂₄ fatty acid are chosen from lauric acid and stearic acid, preferably lauric acid.

9. Composition according to any one of the preceding claims, **characterized in that** the oxyethylenated sorbitan esters are chosen from the monoesters of a C₈-C₂₄ fatty acid and of oxyethylenated sorbitan.

10. Composition according to any one of the preceding claims, **characterized in that** the number of moles of ethylene oxide ranges from 3 to 8 mol of ethylene oxide, and in particular is equal to 4 mol.

11. Composition according to any one of the preceding claims, **characterized in that** the oxyethylenated sorbitan esters are chosen from sorbitan monolaurate oxyethylenated with 4 mol of ethylene oxide (4 EO) and sorbitan monostearate oxyethylenated with 4 mol of ethylene oxide (4 EO).

12. Composition according to any one of the preceding claims, **characterized in that** the oxyethylenated sorbitan ester is sorbitan monolaurate oxyethylenated with 4 mol of ethylene oxide (4 EO).

13. Composition according to any one of the preceding claims, **characterized in that** the oxyethylenated sorbitan ester is present in the composition in proportions ranging from 0.1% to 10% and preferentially from 0.5% to 8% by weight relative to the total weight of the said composition.

14. Composition according to one of the preceding claims, **characterized in that** the plant oils are chosen from sweet almond oil, avocado oil, castor oil, olive oil, jojoba oil, sunflower oil, wheatgerm oil, sesame oil, groundnut oil, grapeseed oil, soybean oil, rapeseed oil, safflower oil, coconut oil, corn oil, hazelnut oil, shea butter, palm oil, apricot kernel oil, liquid jojoba wax, cade oil and beauty-leaf oil, and mixtures thereof.

15. Composition according to one of the preceding claims, **characterized in that** the hydrocarbon-based oils are chosen from liquid paraffin, liquid petroleum jelly and isoparaffins, and mixtures thereof.

16. Composition according to any one of the preceding claims, **characterized in that** the liquid fatty substance is present at a concentration ranging from 0.5% to 3% by weight relative to the total weight of the composition.

17. Composition according to any one of the preceding claims, **characterized in that** it also comprises at least one additional conditioning agent.

18. Composition according to Claim 17, **characterized in that** the additional conditioning agent is chosen from silicones, cationic polymers, solid carboxylic fatty esters and mixtures thereof.

19. Composition according to any one of the preceding claims, **characterized in that** the cosmetically acceptable aqueous medium consists of water or of a mixture of water and of a cosmetically acceptable solvent.

20. Composition according to Claim 19, **characterized in that** the cosmetically acceptable solvent is chosen from C₁-C₄ lower alcohols such as ethanol, isopropanol, tert-butanol or n-butanol; alkylene glycols, for instance propylene glycol; polyol ethers; C₅-C₁₀ alkanes, and mixtures thereof.

21. Composition according to any one of the preceding claims, **characterized in that** it also comprises additives such as anionic, nonionic or amphoteric polymers, thickeners such as acids or electrolytes, opacifiers, nacreous agents, vitamins, provitamins such as panthenol, fragrances, dyes, organic or mineral particles, preserving agents and pH stabilizers.

22. Composition according to any one of the preceding claims, **characterized in that** it is in the form of a hair conditioner, a composition for permanent-waving, relaxing, dyeing or bleaching the hair, or a rinse-out composition to be applied between the two steps of a permanent-waving or hair-relaxing operation.

23. Composition according to any one of the preceding claims, **characterized in that** it is in the form of a rinse-out hair conditioner.

24. Cosmetic process for treating keratin materials, such as the hair, **characterized in that** it consists in applying to the said materials an effective amount of a cosmetic composition according to one of Claims 1 to 23, and optionally rinsing it off.
